# EUROPÄISCHE PATENTSCHRIFT

(11) **EP 2 057 176 B1**
(45) Veröffentlichungstag und Bekanntmachung des Hinweises auf die Patenterteilung: **16.10.2013**
(21) Anmeldenummer: 07801845.4
(22) Anmeldetag: 23.08.2007
(51) Int. Cl.: C07H 21/00

(54) **PROGRAMMIERBARE OLIGONUKLEOTIDSYNTHESE**
PROGRAMMABLE OLIGONUCLEOTIDE SYNTHESIS
SYNTHÈSE PROGRAMMABLE D'OLIGONUCLÉOTIDES

(30) Priorität: 23.08.2006 DE 102006039479
(43) Veröffentlichungstag der Anmeldung: 13.05.2009
(60) Teilanmeldung: 12163204.6
(73) Patentinhaber: Synthetic Genomics, Inc., La Jolla, CA 92037 (US)
(72) Erfinder: STÄHLER, Peer, 68167 Mannheim (DE); CARAPITO, Raphael, 67100 Strasbourg (FR)
(74) Vertreter: Weiss, Wolfgang
(86) Internationale Anmeldenummer: PCT/EP2007/007417
(87) Internationale Veröffentlichungsnummer: WO 2008/022789

(56) Entgegenhaltungen:
- WO-A-00/49142
- RICHMOND K E ET AL: "Amplification and assembly of chip-eluted DNA (AACED): a method for high-throughput gene synthesis" NUCLEIC ACIDS RESEARCH, IRL PRESS LTD., OXFORD, GB, Bd. 32, Nr. 17, 2004, Seiten 5011-5018, XP002344586 ISSN: 0305-1048
- TIAN J ET AL: "Accurate multiplex gene synthesis from programmable DNA microchips" NATURE, NATURE PUBLISHING GROUP, LONDON, GB, Bd. 432, Nr. 7020, 23. Dezember 2004 (2004-12-23), Seiten 1050-1054, XP002371017 ISSN: 0028-0836
- HOOVER DAVID M ET AL: "DNAWorks: an automated method for designing oligonucleotides for PCR-based gene synthesis" NUCLEIC ACIDS RESEARCH, OXFORD UNIVERSITY PRESS, SURREY, GB, Bd. 30, Nr. 10, 15. Mai 2002 (2002-05-15), Seiten e43-1, XP002301503 ISSN: 0305-1048

## Beschreibung

Die Erfindung betrifft Verfahren und Vorrichtungen zur Herstellung von synthetischen Nukleinsäuren.

### Einleitung

Der Bedarf an synthetischen Nukleinsäuren ist durch die Molekularbiologie und die biomedizinische Forschung und Entwicklung hoch. Die Herstellung von synthetischen Nukleinsäuren (DNA, RNA oder deren Analoga) wird überwiegend mit Hilfe von säulengestützten Synthesizern durchgeführt.

Besonders wichtige und weit verbreitete Einsatzgebiete für synthetische Nukleinsäurepolymere sind Primer für die *Polymerase chain reaction* (PCR) (Critical Reviews in Biochemistry and Molecular Biology 26 (3/4), 301-334, 1991) und die Sequenziermethode nach Sanger (Proc. Nat. Acad. Sci. 74, 5463-5467, 1977).

Synthetische DNA spielt auch für die Herstellung von synthetischen Genen eine Rolle. Beschrieben werden Methoden der Gensythese als Beispiel in US 6 586 211 B1, in PCT/EP2004/013131, in WO 00/13017 A2, in S. Rayner et al., PCR Methods and Applications 8 (7), 741-747, 1998, in WO 90/00626 A1, in EP 385 410 A2, in WO 94/12632 A1, in WO 95/17413 A1, in EP 316 018 A2, in EP 022 242 A2, in L. E. Sindelar and J. M. Jaklevic, Nucl. Acids Res. 23 (6), 982-987, 1995, in D. A. Lashkari, Proc. Nat. Acad. Sci. USA 92 (17), 7912-7915, 1995, in D.M. Hoover und J. Lubkowski, Nucleic Acids Res. 30 (10), e43 (2002) und in WO 99/14318 A1.

Zwei weitere Anwendungsfelder mit steigendem Bedarf sind die Herstellung von Mikroarrays oder Biochips aus Oligonukleotid-Sonden (1. Nature Genetics, Vol. 21, Supplement (gesamt), Jan. 1999, 2. Nature Biotechnology, Vol. 16, 981-983, Okt. 1998, 3. Trends in Biotechnology, Vol. 16, 301-306, Jul. 19989) und die Herstellung von interferierender RNA (iRNA oder RNAi) für die Modulation der Genexpression in Zielzellen (PCT/EP01/13968).

Die genannten Anwendungsgebiete der Molekularbiologie liefern wertvolle Beiträge in der Wirkstoff-Entwicklung, der Wirkstoff-Produktion, der kombinatorischen Biosynthese (Antikörper, Effektoren wie Wachstumsfaktoren, Neurotransmitter etc.), in der Biotechnologie (z.B. Enzymdesign, Pharming, biologische Herstellungsverfahren, Bioreaktoren etc.), in der molekularen Medizin im Tissue Engineering, in der Entwicklung und Anwendung neuer Materialien (z.B. Werkstoffe wie Spinnenseide und Perlmutt), in der Entwicklung und Anwendung von Diagnostika (Mikroarrays, Rezeptoren und Antikörper, Enzymdesign etc.) oder in der Umwelttechnik (spezialisierte oder maßgeschneiderte Mikroorganismen, Produktionsverfahren, Sanierung, Sensoren etc.). Die Anwendung des erfindungsgemäßen Verfahrens kann somit in allen diesen Bereichen erfolgen.

### Stand der Technik

Die am weitesten verbreitete Methode zur Herstellung von synthetischen Nukleinsäuren basiert auf Grundlagenarbeiten von Caruthers und wird als Phosphitamid-Methode beschrieben (M. H. Caruthers, Methods in Enzymology 154, 287-313, 1987). Die Sequenz der entstehenden Moleküle kann dabei durch die Syntheseabfolge gesteuert werden. Andere Verfahren, wie z.B. die H-Phosphonat-Methode, dienen dem gleichen Zweck des sukzessiven Aufbaues eines Polymers aus seinen Untereinheiten, haben sich aber nicht so weit durchsetzen können wie die Methode nach Caruthers.

Um das chemische Verfahren der Polymersynthese aus Untereinheiten automatisieren zu können, werden meistens feste Phasen verwendet, an denen die wachsende Molekülkette verankert ist. Sie wird erst nach Fertigstellung der Synthese abgespalten, wozu ein geeigneter Linker zwischen dem eigentlichen Polymer und der festen Phase erforderlich ist. Die Methode verwendet zur Automatisierung in der Regel feste Phasen in Form aktivierter Partikel, die in eine Säule gefüllt werden, z.B. *controled pore glass* (CPG). Solche festen Phasen tragen in der Regel nur eine definiert entnehmbare Art von Oligo mit einer programmierten Sequenz. Die Zugabe der einzelnen Synthesereagenzien erfolgt nun in einer steuerbaren Art und Weise in einem Automaten, der vor allem die automatisierte Zugabe der einzelnen Reagenzien zur festen Phase sicherstellt. Die Menge an synthetisierten Molekülen ist durch die Menge des Trägermaterials und die Größe der Reaktionsansätze steuerbar. Diese Mengen sind für die oben genannten molekular-biologischen Verfahren entweder ausreichend oder sogar zu hoch (z.B. bei PCR-Primern). Eine gewisse Parallelisierung zur Erzeugung einer Vielzahl unterschiedlicher Sequenzen wird durch Anordnung von mehreren Säulen in einem apparativen Aufbau erreicht. So sind dem Fachmann Geräte mit 96 parallelen Säulen bekannt.

Eine Variante und Weiterentwicklung für die Herstellung von synthetischen Nukleinsäuren ist die *in situ* Synthese von Mikroarrays (Array-Anordnung der Nukleinsäuren in einer Matrix). Diese wird auf einem Substrat durchgeführt, das durch die Synthese mit einer Vielzahl unterschiedlicher Sequenzen beladen wird. Der große Vorteil der *in situ* Syntheseverfahren für Mikroarrays ist die Bereitstellung einer Vielzahl von Oligomeren unterschiedlicher und definierter Sequenz an adressierbaren Lokationen auf einem gemeinsamen Träger. Die Synthese greift dabei auf ein überschaubares Set aus Einsatzstoffen zurück (bei DNA-Mikroarrays in der Regel die 4 Basen A, G, T und C) und baut aus diesen beliebige Sequenzen der Nukleinsäurepolymere auf.

Die Abgrenzung der einzelnen Molekülspezies kann zum einen durch getrennte fluidische Kompartimente bei der Zugabe der Syntheseeinsatzstoffe erfolgen, wie es z.B. in der so genannten *in situ* Spotting Methode oder Piezoelektrischen Techniken der Fall ist, die auf der Tintenstrahldrucktechnik beruht (A. Blanchard, in Genetic Engineering, Principles and Methods, Vol. 20, Ed. J. Sedlow, 111-124, Plenum Press; A. P. Blanchard, R. J. Kaiser, L. E. Hood, High-Density Oligonucleotide Arrays, Biosens. & Bioelectronics 11, 687, 1996).

Eine alternative Methode ist die ortsaufgelöste Aktivierung von Syntheseplätzen, was durch selektive Belichtung, durch selektive oder ortsaufgelöste Generierung von Aktivierungsreagenzien (Entschützungsreagenzien) oder durch selektive Zugabe von Aktivierungsreagenzien (Entschützungsreagenzien) möglich ist.

Beispiele für die bisher bekannten Verfahren für die *in situ* Synthese von Mikroarrays sind
- die photolithographische lichtgestützte Synthese (McGall, G. et al; J. Amer. Chem. Soc. 119; 5081-5090; 1997),
- die projektorbasierte lichtgestützte Synthese (PCT/EP99/06317),
- die fluidische Synthese mittels physischer Trennung der Reaktionsräume (dem Fachmann bekannt aus den Arbeiten von Prof. E. Southern, Oxford, UK, und von Firma Oxford Gene Technologies, Oxford, UK),
- die indirekte projektorbasierte lichtgesteuerte Synthese mittels lichtaktivierter Photosäuren und geeigneten Reaktionskammern oder physisch getrennten Reaktionsräumen in einem Reaktionsträger,
- die elektronisch induzierte Synthese mittels ortsaufgelöster Entschützung an einzelnen Elektroden auf dem Träger unter Nutzung einer durch die Elektroden induzierten Entstehung von Protonen (bekannt u.a. durch die Produkte der Firma Combimatrix) und
- die fluidische Synthese mittels ortsaufgelöster Deposition der aktivierten Synthese-Monomere (bekannt aus A. Blanchard, in Genetic Engineering, Principles and Methods, Vol. 20, Ed. J. Sedlow, 111-124, Plenum Press; A. P. Blanchard, R. J. Kaiser, L. E. Hood, High-Density Oligonucleotide Arrays, Biosens. & Bioelectronics 11, 687, 1996).

Verfahren zur Herstellung von synthetischen Nukleinsäuren, insbesondere Nukleinsäuredoppelsträngen an einem gemeinsamen festen Träger sind außerdem aus WO 00/49142 und WO 2005/051970 bekannt.

### Gegenstand der Erfindung

Bereitgestellt werden soll ein Verfahren zur Herstellung von Nukleinsäuredoppelsträngen, umfassend die Schritte:
(a) Herstellen einer Vielzahl von unterschiedlichen Nukleinsäurefragmenten durch Festphasensynthese, vorzugsweise an verschiedenen Positionen eines gemeinsamen festen Trägers, und
(b) Zusammenfügen von mindestens zwei Nukleinsäurefragmenten aus (a) durch eine spezifische Hybridisierungsreaktion zwischen Überlappungsbereichen zueinander komplementärer Abschnitte der Nukleinsäurefragmente,
wobei mindestens einige Nukleinsäurefragmente, die einen AT-Gehalt größer als 50 % aufweisen, in einer gegenüber anderen Fragmenten gesteigerten Menge für Schritt (b) eingesetzt werden.

Die Zusammenfügung der Nukleinsäurefragmente erfolgt durch eine spezifische Hybridisierungsreaktion zwischen Überlappungsbereichen zueinander komplementärer Abschnitte der Nukleinsäurefragmente, wobei längere synthetische doppelsträngige Nukleinsäuren erhalten werden. Die, einzelnen, zum Aufbau längerer Nukleinsäuren verwendeten Sequenzabschnitte haben vorzugsweise eine Länge von 20-100 oder 20-300 Nukleotidbausteinen, bevorzugt von 25-50 oder 25-100 Nukleotidbausteinen, beispielsweise etwa 30 Nukleotidbausteinen. Die Sequenzabschnitte werden dabei vorzugsweise so gewählt, dass sie mit einem Sequenzabschnitt des Gegenstrangs der zu synthetisierenden komplementären Nukleinsäure zumindest teilweise überlappen, so dass der zu synthetisierende Nukleinsäurestrang durch Hybridisierung einzelner Sequenzabschnitte aufgebaut werden kann. In einer alternativen Ausführungsform werden die Sequenzabschnitte vorzugsweise so gewählt, dass die Sequenzabschnitte auf beiden Strägen der zu synthetisierenden Nukleinsäure vollständig überlappen, demnach zur Herstellung eines soweit vollständigen Doppelstranges nur noch die kovalente Verknüpfung des Phosphordiester-Rückgrates notwendig ist. Die Länge der Komplementärbereiche bzw. Überlappungen zwischen einzelnen Fragmenten beträgt z.B. 10-50 oder 10-100 Nukleotidbausteine, vorzugsweise 12-25 oder 20-80 Nukleotidbausteine, besonders bevorzugt etwa 15-20 Nukleotidbausteine und am stärksten bevorzugt etwa 15 oder etwa 20 Nukleotidbausteine. Wenn der Überlappungs- bzw. Komplementaritätsbereich zwischen zwei Nukleinsäurefragmenten einen einen AT-Gehalt > 50 %, vorzugsweise einen AT-Gehalt > 60 %, besonders bevorzugt einen AT-Gehalt > 65 % aufweist, ist die Bindungskonstante im Vergleich zu GC-reicheren Sequenzen niedriger. Demzufolge kann die Hybridisierung zwischen diesen Fragmenten aus thermodynamischen Gründen vergleichsweise wenig effektiv sein. Dies kann einen Einfluss auf die Zusammenlagerung von 2 oder mehr Fragmenten ausüben. Eine mögliche sequenzabhängige Folge ist eine Verringerung der Ausbeute von Nukleinsäuredoppelsträngen mit der korrekten Ziel-Sequenz.

Ein Ziel des erfindungsgemäßen Verfahrens ist die Beineinflussung der thermodynamischen Zusammenhänge während der Zusammenlagerung aus 2 oder mehr Fragmenten durch Steuerung oder durch Steuerung und Kontrolle der Mengenverhältnisse der Fragmente in einem Reaktionsansatz, um die Ausbeute an korrekten Nukleinsäuredoppelsträngen zu verbessern. Hierbei werden insbesondere die thermodynamischen Parameter in einer Reaktion zur Bindung von mindestens 2 Nukleinsäurefragmenten aneinander moduliert. Unter Modulation der thermodynamischen Parameter wird insbesondere verstanden, dass die Bindung der beiden Nukleinsäurefragmente aneinander, die dem Massenwirkungsgesetz unterliegt, verbessert wird. Die Modulation der thermodynamischen Parameter in der Reaktion ist die Steuerung der Mengenverhältnisse einzelner Nukleinsäurefragmente durch Verwendung höherer Mengen von Nukleinsäurefragmenten, die einen AT-Anteil > 50 % aufweisen. Wenn mindestens einige Nukleinsäurefragmente zur Modulation ihrer thermodynamischen Parameter in der Reaktion von mindestens 2 Nukleinsäurefragmenten in einer gegenüber anderen Fragmenten gesteigerten Menge eingesetzt werden, so kann dies bewirkt werden, indem mindestens einige Nukleinsäurefragmente, die einen AT-Gehalt > 50 % aufweisen, in einer gegenüber anderen Fragmenten gesteigerten Menge eingesetzt werden.

Durch Steuerung der Mengenverhältnisse einzelner Nukleinsäurefragmente durch Verwendung höherer Mengen von Nukleinsäurefragmenten, die einen AT-Anteil > 50 % aufweisen, kann die Ausbeute an korrekten Hybridisierungsprodukten und somit auch die Ausbeute an korrekten Nukleinsäuredoppelsträngen verbessert werden. Dabei kann die Menge der Population der entsprechenden Nukleinsäurefragmente um vorzugsweise ≥ 10 %, besonders bevorzugt ≥ 50% oder noch mehr, z.B. bis um den Faktor 100 oder 1000 mehr gegenüber anderen Nukleinsäurefragmenten mit einem AT-Anteil ≤ 50 % gesteigert werden.

Außer dem AT-Anteil gibt es noch weitere Parameter, die einen Einfluss auf die Ausbeute an Zielsequenzen haben. Hierzu gehört die unterschiedliche Syntheseeffizienz der einzelnen Fragmente oder Oligonukleotide während der Verlängerung im Syntheseprozess. Es ist dem Fachmann z.B. bekannt, dass der Baustein G bei Phosphoramidit-Verfahren mit geringerer Ausbeute an den zu verlängernden Polymerstrang koppelt als die anderen NukleotidBausteine. Außerdem sind dem Fachmann empirisch sichtbare, aber nicht in jedem Fall bereits vorhersagbare Abhängigkeiten der Syntheseeffizienz von der gesamten Sequenz des Polymerstranges bekannt. Hierzu zählt z.B. die Synthese von mehreren G Bausteinen nacheinander.

Diese Abweichungen in der Verfügbarkeit oder Kinetik einzelner Fragmente für die Zusammenlagerung von 2 oder mehr Fragmenten zu einer Zielsequenz können ebenfalls durch Steuerung oder Steuerung und Kontrolle (Regelung) der Mengenverhältnisse beeinflusst werden. Dabei kann die Abweichung berechenbar und gut bekannt oder lediglich aus der Empirie bekannt und im Experiment beobachtet sein. Dementsprechend kann das erfindungsgemäße Verfahren z.B. iterativ unter Messung des Ergebnisses, also z.B der Ausbeute an Zielsequenz, optimiert werden. Eine Ausführungsform der Erfindung ist die Nutzung einer speicherprogrammierbaren Einrichtung, um auf Grundlage bekannter Gesetzmäßigkeiten für neue Fragmente-Sequenzen und Zielsequenzen die vorhergesagte optimale Zusammensetzung der Mengenverhältnisse zu steuern. Dies kann in der Umsetzung mittels eines Computers oder ähnlicher Steuerungstechniken erfolgen. Dabei können Einflussgrößen und Stellparameter in einer Datenbank erfasst werden, die in einer Ausführungsform in einer speicherprogrammierbaren Einrichtung enthalten ist und bei der Steuerung der Synthese mittelbar oder unmittelbar eingesetzt wird.

Die Reaktionsprodukte einer Bibliothekssynthese zeichnen sich durch große Vielfalt der Sequenzen aus, die während des Synthesevorgangs frei wählbar programmiert werden können. Ein Zahlenbeispiel illustriert die Vielfalt einer solchen Bibliothek. Ein Mikroarray aus dem Geniom®-System, bei dem die Nukleinsäure-Molekülpopulationen auf einzelnen Syntheseplätzen in einem speziellen mikrofluidischen Träger synthetisiert werden, kann zum Beispiel mit Stand 2006 bis zu 60.000 frei wählbare Oligonukleotide mit einer Sequenz von bis zu 60 Nukleotiden synthetisieren. Die Geräte synthetisieren die Nukleinsäuren ortsaufgelöst unter Nutzung eines projektorbasierten Verfahrens (siehe z.B. WO 00/13018 oder WO 00/13017).

Zweck des verbesserten Verfahrens ist die Bereitstellung von Nukleinsäuren mit hoher und rationell programmierbarer Diversität der Sequenzen und steuerbaren Mengenverhältnissen der einzelnen Sequenz-Vertreter oder Fragmente (Bestandteile der Bibliothek) für in einem nächsten Schritt nachfolgende Verfahren.

Beispiele für nachfolgende Verfahren, für die die Erfindung verwendet werden kann, sind:
- die Herstellung von Nukleinsäurefragmenten als Primer für Primer Extension Methoden, Strand Displacment Amplifikation, Polymerase Chain Reaction, Site Directed Mutagenesis oder Rolling Circle Amplifikation,
- Herstellung von synthetischen Genen, Genfragmenten, Gen-Clustern, Gen-Fähren, Gen-Vektoren, Chromosomen, Genomen, optimierten Genomen, minimalen Gen-Clustern, minimalen Genomen, vollsynthetischen Genomen oder von Mischungen mit gezielten oder randomisierten Varianten von synthetischen Genen, Genfragmenten, Gen-Clustern, Gen-Fähren, Gen-Vektoren, Chromosomen, Genomen, optimieren Genomen, minimalen Gen-Clustern, minimalen Genomen, vollsynthetischen Genomen,
- Genexpressions-Modulation mittels RNAi oder Antisense-Methoden, wobei noch eine Abschrift von der erzeugten Nukleinsäure oder den Nukleinsäuren durch eine RNA Polymerase vorgesehen sein kann,
- Herstellung, Extraktion, Aufreinigung, Isolation oder Vorbereitung von Analyten (sample preparation) für die logisch nachgeordnete Analyse durch Mikroarrays, durch Sequenzierverfahren, durch parallele Sequenzierverfahren, durch Amplifikationsverfahren (Strand Displacment Amplifikation, Polymerase Chain Reaction oder Rolling Circle Amplifikation) oder Analyse in einer Gelelektrophorese,
- RNA-Bibliotheken mit z.B. 2 oder mehr Sequenzen für die Translation in *vitro* oder *in vivo*,
- Klonierung der erzeugten Nukleinsäuren alleine oder in Kombination mit weiteren Sequenzen mittels Vektoren oder Plasmiden,
- Herstellung von Minimalgenomen, optimierten Genomen, reduzierten Genomen, gemischten Genomen verschiedener Spezies, wobei der gesamte geplante Bestand des Genomes oder ein Teil davon durch die synthetischen Nukleinsäuren kodiert werden kann,
- Herstellung von Zielorganismen mit permanent oder temporär integrierten, transformierten, transfizierten oder anderweitig eingebrachten synthetischen Genen, Genfragmenten, Gen-Clustern, Gen-Fähren, Gen-Vektoren, Chromosomen, Genomen, optimierten Genomen, minimalen Gen-Clustern, minimalen Genomen, vollsynthetischen Genomen oder von Mischungen mit gezielten oder randomisierten Varianten von synthetischen Genen, Genfragmenten, Gen-Clustern, Gen-Fähren, Gen-Vektoren, Chromosomen, Genomen, optimieren Genomen, minimalen Gen-Clustern, minimalen Genomen, vollsynthetischen Genomen,
- Herstellung der im vorhergehenden Punkt beschriebenen Zielorganismen für die Verbesserung, Veränderung oder Reduktion eines in dem Zielorganismus anfallenden Naturstoffes, z.B. einer Aminosäure-Kette, eines Proteins, einer organischen Substanz, eines Kohlenwasserstoffs, eines Pharmakons oder einer Vorstufe davon, einer Nukleinsäure, eines Pheromons, oder einer anderen Substanz für die Bereitstellung eines von Menschen genutzten Gutes oder für eine Herstellung einer Substanz, die anschließend einer weiteren Nutzung zugeführt wird,
- Ligation der Nukleinsäuren in Vektoren, YACs, BACs, Chromosomen oder Plasmide,
- Validierung oder Test von Hybridisierungs-Assays und zugehörigen Reagenzien und Kits mittels der erzeugten Nukleinsäurepolymeren in den Bereichen Mikroarrays, Biochips, Dot blots, Southern- oder Northern-Blots, Bead-Arrays, Serial Analysis of Gene Expression (SAGE), PCR , real time PCR,
- Referenz- oder Kalibrier-Verfahren oder Verfahrensschritte innerhalb von Assays aus den Bereichen Mikroarrays, Dot blots, Southern- oder Northern-Blots, Bead-Arrays, Serial Analysis of Gene Expression (SAGE), PCR, real time PCR,
- Herstellung von bindenden Agenzien wie Aptameren und Ribozymen sowie indirekte Herstellung über die Translation *in vivo* oder *in vitro* von Peptiden, Proteinen, Antikörpern, Antikörperfragmenten, Antikörper analog wirkenden Peptiden, Antikörper-analog wirkenden Proteinen,
- Herstellung über die Translation *in vivo* oder *in vitro* von Glykoproteinen, Proteoglykanen oder Komplexen mit wahlweise Peptid-Anteil, ProteinAnteil, RNA-Anteil oder DNA-Anteil, wie Ribosomen oder Proteasomen.

### Ausführliche Beschreibung von Erfindung und Ausführungsformen

Bevorzugte Verfahren zur Herstellung von synthetischen Nukleinsäuren aus einem festen Träger sind aus WO 00/49142 und WO 2005/051970 bekannt. Auf den Inhalt dieser Dokumente wird ausdrücklich Bezug genommen.

Vorzugsweise erfolgt im erfindungsgemäßen Verfahren die Herstellung der synthetischen Nukleinsäuren dadurch, dass eine Vielzahl von unterschiedlicher Nukleinsäurefragmente an verschiedenen Positionen eines gemeinsamen, festen Trägers synthetisiert wird. Vorzugsweise umfasst die Synthese der Nukleinsäurefragmente einen Aufbau aus Nukleotidbausteinen mittels nass- und/oder fotochemischer Verfahren auf dem Träger, anschließendes Ablösen der Nukleinsäurefragmente und Assemblieren der Fragmente zu dem gewünschten Nukleinsäuredoppelstrang. Weiterhin kann die Synthese Amplifikationsschritte umfassen, bei denen die synthetisierten Nukleinsäurefragmente oder/und daraus gebildete, gegebenenfalls doppelsträngige Zwischenprodukte einer Amplifikation, z.B. einer PCR, unterzogen werden. Hierzu können Nukleotidbausteine und ein Enzym, das eine Amplifikation bewirkt, zugeführt werden. Amplifikationen können auf dem Träger, d.h. vor oder/und nach Ablösung der Nukleinsäurefragmente, oder/und nach Elution vom Träger erfolgen.

Der Träger kann ausgewählt werden aus flachen Trägern, porösen Trägem, Reaktionsträgem mit Elektroden, Reaktionsträgern mit Partikeln oder Beads, mikrofluidischen Reaktionsträgern, die gegebenenfalls Oberflächenmodifikationen wie Gele, Linker, Spacer, Polymere, amorphe Schichten oder/und 3D-Matrices aufweisen, und Kombinationen der zuvor genannten Träger. Vorzugsweise ist der Träger ein mikrofluidischer Träger.

Die Nukleinsäurefragmente werden vorzugsweise durch orts- oder/und zeitaufgelöste *in situ* Synthese auf dem Träger erzeugt, beispielsweise durch orts- oder/und zeitaufgelöste Belichtung mittels einer programmierbaren Lichtquellenmatrix. Die ort- oder/und zeitaufgelöste Synthese kann in einem mikrofluidischen Träger mit einem oder mehreren fluidischen Reaktionsräumen und einem oder mehreren Reaktionsbereichen innerhalb eines fluidischen Reaktionsraums erfolgen.

Unterschiedliche Mengen von für die Assemblierung verwendeten Nukleinsäurefragmentspezies können durch Verwendung von mehreren Bereichen und/oder größeren Bereichen für die Synthese der jeweiligen Nukleinsäurefragmente auf dem Träger erzeugt werden. Ein entsprechend modifizierter Träger ist auch Gegenstand der Erfindung.

Eine weitere Ausführungsform der Erfindung besteht darin, die Assemblierung von Nukleinsäurefragmenten zu Nukleinsäuredoppelsträngen in mehreren Schritten durchzuführen. In einem ersten Schritt werden dabei die auf dem Träger synthetisierten Nukleinsäurefragmente am 5'- oder/und 3'-Ende mit einer oder mehreren generischen Primersequenzen von vorzugsweise 10-20 oder 10-100 Basen, besonders bevorzugt von 10-30 Basen, noch stärker bevorzugt etwa 15 Basen versehen, wobei die Primersequenzen so gewählt sind, dass eine Amplifikation direkt für das einzelne Fragment, für einen Teil aller Fragmente in einem Gemisch, für alle Fragmente in einem Gemisch oder nach Hybridisierung von zwei oder mehr Nukleinsäurefragmenten mit teilweisen komplementärer Sequenz möglich ist. Nach Abspalten der mit Primem versehenen Nukleinsäurefragmente vom Träger und gegebenenfalls nach Elution aus dem Träger und gegebenenfalls einer Hybridisierung von Fragmentpaaren mit komplementärer Sequenz erfolgt eine anschließende Amplifikation, z.B. durch PCR, durch Zugabe entsprechender Primer. In der Amplifikationsreaktion entstehen Nukleinsäurefragmente, die an ihren Enden die generische Primersequenz enthalten. Nach Abspaltung der Primersequenz, z.B. mittels Restriktionsendonukleasen, können die resultierenden Nukleinsäurefragmente weiteren Amplifikationszyklen unterzogen werden, um einen Nukleinsäuredoppelstrang zu erzeugen.

Der durch Synthese von Fragmenten und deren anschließende Assemblierung erzeugte Nukleinsäuredoppelstrang wird in einer Ausführungsform in einen Vektor, z.B. in ein Plasmid, eingebaut und in eine geeignete Wirtszelle, z.B. eine Bakterienzelle, überführt.

Die Herstellung der Nukleinsäurepolymere bietet an mehreren Stellen des Verfahrens die Möglichkeit, mit bekannten Methoden in die Reaktionsprodukte Modifikationen oder Markierungen einzuführen. Hierzu zählen markierte Nukleotide, die z.B. mit Haptenen oder optischen Markem, wie Fluorophoren und Lumineszenz-Markem, modifiziert sind, markierte Primer oder Nukleinsäure-Analoga mit besonderen Eigenschaften, wie z.B. besondere Schmelztemperatur oder Zugänglichkeit für Enzyme. Ausführungsformen der Erfindung können dadurch folgende Funktionen und Methoden einschließen und die zugehörigen Laborprozesse ermöglichen:
- Markierung der Oligonukleotide, der Fragmente, von daraus aufgebauten kovalent oder nicht-kovalent verbundenen Hybridsträngen sowie der Zielsequenzen, für eine direkte oder indirekte Erfassung mit einem Messgerät, z.B. einem optischen, magnetischen, elektrischen oder chemilumineszenten Messgerät oder Messverfahren,
- Markierung der Oligonukleotide, der Fragmente, von daraus aufgebauten kovalent oder nicht-kovalent verbundenen Hybridsträngen sowie der Zielsequenzen, für eine direkte oder indirekte Erfassung durch andere molekulare Strukturen wie z.B. Enzyme, Rezeptoren, Proteine,
- Isolation oder Extraktion der Oligonukleotide, der Fragmente, von daraus aufgebauten kovalent oder nicht-kovalent verbundenen Hybridsträngen sowie der Zielsequenzen,
- Bindung der Oligonukleotide, der Fragmente, von daraus aufgebauten kovalent oder nicht-kovalent verbundenen Hybridsträngen sowie der Zielsequenzen mittels der Haptene oder anderer in einer molekularen Erkennungsreaktion einsetzbaren Bausteine, einer funktionellen Gruppe oder einer Modifikation,
- Detektion der Oligonukleotide, der Fragmente, von daraus aufgebauten kovalent oder nicht-kovalent verbundenen Hybridsträngen sowie der Zielsequenzen,
- Abbau, selektive Trennung, Öffnung, Degradation oder enzymatischer Verdau der Oligonukleotide, der Fragmente, von daraus aufgebauten kovalent oder nicht-kovalent verbundenen Hybridsträngen sowie der Zielsequenzen,
- Anlagerung der Oligonukleotide, der Fragmente, von daraus aufgebauten kovalent oder nicht-kovalent verbundenen Hybridsträngen sowie der Zielsequenzen an eine Zielstruktur oder an Zielstrukturen, z.B. in einem oder auf einem Reaktionsträger, auf einem Glas-Objektträger, in einem Reaktionsgefäß, an einer Zielorganelle, an einer Zielzelle, an einem Zielorgan, an einem Organismus, an einer Oberfläche, an einer biologischen Oberfläche, an einem molekularen Komplex wie z.B. einem Chromosom, Viruspartikel oder Proteinkomplex.

Im Folgenden ist eine beispielhafte Ausführungsform der Erfindung und Ablauf des Verfahrens unter Nutzung der GENIOM^{®}-Plattform dargestellt:
1. Design eines Mikroarrays aus 6.000 verschiedenen 30-40meren, insbesondere 30meren oder 40meren Nukleinsäurefragmenten in einem GENIOM^{®}-Gerät. Bei dem Design des Mikroarrays wird die Anzahl der Synthesespots pro Sequenz zwischen 1 und 100 (in einfachen Schritten) gewählt, wobei Nukleinsäurefragmente (Oligos) mit geprüfter oder vorhergesagter schwächerer Bindung relativ zu anderen Sequenzen, die später an einer Zusammenlagerung teilnehmen, mit einer um vorzugsweise 50 % oder mehr größeren Anzahl an Synthesespots vertreten sind, als mindestens eine andere Oligo-Sequenz, um den prozentualen Anteil im Gemisch zu erhöhen und so die entsprechenden Hybride gegenüber den stärker bindenden anderen Sequenzen zu fördern.
2. Anfügen einer generischen Primer-Sequenz von 10-15, insbesondere 10 oder 15 Basen an allen 6.000 Oligos, insbesondere jeweils an beiden Enden, so dass alle Sequenzen z.B. 60 Basen umfassen. Die Primersequenz kann so gewählt werden, dass eine PCR-Reaktion durch Hybridisierung von jeweils zwei Oligos mit komplementärer Sequenz möglich ist.
3. Herstellen des Mikroarrays anhand des Designs aus 1. und 2. durch Synthese im GENIOM^{®}-Gerät.
4. Abspalten und Elution der Oligos aus dem Reaktionsträger.
5. PCR der Library unter Zugabe eines zu den unter 2 angefügten PrimerSequenzen passenden Primerpaares. In der PCR entstehen z.B. 60mere, die jeweils ein Insert von z.B. 40 Basen mit wahlfreier Sequenz und einheitliche Sequenzen an beiden Enden tragen können.
6. Abspalten der Primer-Sequenz.
7. Inkubation der resultierenden 30-40mer Library bzw. der 30mer oder 40mer Library mit neuen PCR-Reagenzien unter Zugabe eines Primerpaares, welches z.B. nur an im Hybrid um mindestens 2 Fragmente auseinander liegenden Sequenzen bindet und somit zu einer "nested PCR" eines längeren Fragments führt (eine direkte Zusammenlagerung und PCR Amplifikation eines synthetischen Gens ist dem Fachmann durch Publikationen bekannt).
8. Weitere Bearbeitung oder Lagerung des resultierenden Vervielfältigungsproduktes (Amplikon). In einer Ausführungsform wird das Amplikon unter Nutzung eines Plasmids in Bakterien kloniert und nach Anwachsen von Klonen eine Anzahl von 10 Klonen oder 10 Inserts in den Klonen sequenziert. Das sequenzgeprüfte synthetische Gen steht zur Verfügung.

Im Stand der Technik ist bekannt, dass die Nutzung von unterschiedlichen Mengenverhältnissen der einzelnen Oligonukleotide zueinander für eine Zusammenlagerung von synthetischen Genen die Rate an korrekten Sequenzen erhöht (Gao, X et al., Nucleic Acids Research, 2003, Vol 31; No. 22; P:143). Die Stöchiometrie der Oligonukleotide hat einen Einfluss auf die thermodynamischen Parameter und wirkt sich durch das Massenwirkungsgesetz aus.

In der bevorzugten Ausführungsform und in einer Reihe der eingangs beschriebenen Herstellverfahren für die Herstellung von Mikroarrays *in situ* kann das Design, d.h. die konkrete Beladung und Zuweisung von Fläche und Lokation auf dem Reaktionsträger für eine einzelne Oligonukleotid-Spezies, flexibel gewählt werden und somit auch die Menge der einzelnen Oligonukleotide. Grundsätzlich ist dies mit allen dem Fachmann bekannten und eingangs aufgelisteten *in situ* Syntheseverfahren möglich. Besonders bevorzugt für die Ausführungsformen der Erfindung sind solche Verfahren, die flexibel programmiert werden können und keine Änderung physischer Teile im Produktionsaufbau erfordern. Hier sind z.B. vor allem die ink jet Spotting Verfahren, die Projektions-Verfahren und die elektrochemische Methode nach Combimatrix vorteilhaft.

Die Menge der einzelnen Oligonukleotide ist entsprechend nach Ablösen und Elution der Oligos auch in dem Pool an diversen Oligo-Spezies in Lösung steuerbar. Bei Verwendung von Projektionstechnologie für die Synthese auf dem Reaktionsträger kann die Mengenverteilung über die Anzahl der Mikrospiegel oder Projektionselemente (Belichtungspixel) eingestellt werden. Dem Fachmann ist aus diesem Beispiel ersichtlich, dass auch bei anderen Verfahren in Analogie das Design die Mengen bestimmt. So wird zum Beispiel bei einem photolithographischen Verfahren über die Fläche der Syntheseplätze die Menge bestimmt, bei einer indirekten, über Photosäuren arbeitenden Methode über die Anzahl an physisch abgetrennten Reaktionsplätzen, die für eine Sequenz verwendet werden.

Die Einstellung und Steuerung der Mengenverhältnisse kann durch einen Anwender oder durch eine Software erfolgen. Durch eine entsprechende Software können Vorgaben für die Einstellung der Mengenverhältnisse programmiert werden. Dadurch kann der Prozess an bestimmten Stellen automatisiert werden. Die Vorgaben können aus theoretischen Modellen, Bioinformatik, Sequenz-Vergleichen, empirischen Daten oder Informationen in Datenbanken abgeleitet sein.

Für die empirische Bestimmung und für die Ermittlung der optimalen Anzahl an Syntheseeinheiten pro Sequenz, in einer bevorzugten Ausführungsform an Mikrospiegeln in einer Projektionseinheit, kann eine Hybridisierungsreaktion auf einem Mikroarray verwendet werden. Dazu werden die in Lösung befindlichen ausgewählten Oligos auf einen Mikroarray hybridisiert, der solche Sequenzen enthält, wie sie für die Zusammenlagerung als jeweils komplementäre Gegenparts auf den jeweils korrespondierenden Oligos vorgesehen sind. Diese Analyse simuliert die Zusammenlagerungsreaktion. Das Ergebnis kann in einer Ausführungsform durch Fluoreszenzmarker, die direkt oder indirekt an den in Lösung befindlichen Oligos befestigt sind, ermittelt werden. Die Signale von einzelnen Analyse-Spots kann man dann relativ zueinander auswerten oder quantifizieren. Als Ergebnis dieser Analyse kann das Mengenverhältnis durch Veränderung des Synthese-Design angepasst werden.

Die Anpassung der Mengenverhältnisse verbessert die Bindungsbedingungen für Oligos mit erhöhter Menge in dem Gemisch, und inhärent vergleichsweise niedrigere Bindungsstärken werden ausgeglichen. Die Wahrscheinlichkeit für die korrekte Einlagerung in das Gesamtensemble von Oligos, die an der Reaktion teilnehmen, wird für alle Oligos equilibriert.

Eine vergleichsweise niedrigere Bindungsstärke eines Oligos mit einer passenden Sequenz auf einem zweiten Oligo kann neben weiteren Parametern durch die Basenzusammensetzung, vor allem durch den AT-Anteil (wenn nur natürliche Basen verwendet werden), durch die Neigung zu Sekundärstrukturen oder durch weitere Interaktionen mit anderen Oligos in dem Gemisch verursacht sein.

In einer Ausführungsform ist die Vervielfältigung der abgelösten Oligonukleotide Bestandteil des Verfahrens.

Die Oligonukleotide oder ein Teil davon können mit generischen 5'- und/oder 3'-Sequenzen, angefügt an die Sequenz der herzustellenden Nukleinsäure, synthetisiert werden, so dass anschließend eine Amplifikation der Oligonukleotide oder eines Teils davon erfolgen kann. Die Amplifikations- bzw. Primersequenzen sind komplementär zu entsprechenden Amplifikationsprimern und können eine oder mehrere Spaltungsstellen, vorzugsweise TypII-Spaltungsstellen, enthalten. Diese Spaltungsstellen ermöglichen eine Abspaltung der Primersequenzen nach der Amplifikation, z.B. durch PCR. Auf einem Träger können mehrere Paare von Ampliflkationsprimersequenzen verwendet werden, um eine Multiplex-Amplifikation, z.B. PCR, in einer Oligonukleotidbibliothek zu ermöglichen. Dies bedeutet, dass Subpopulationen der Oligonukleotidfragmente spezifische, aber unterschiedliche Amplifikationssequenzen oder Paare von Amplifikationssequenzen enthalten können. Die Amplikons können aufgereinigt oder direkt für die Amplifikationsreaktion, z.B. für die PCR, eingesetzt werden.

In dieser Ausführungsform kann das Verfahren zur selektiven Amplifikation einer Subpopulation der von Träger stammenden Sequenzen verwendet werden. Dabei wird auch die Vervollständigung von Nukleinsäurefragmenten ermöglicht, die nach der Synthese nicht in Volllängenform waren. Die Amplifikationen können direkt in den Mikrokanälen des Trägers oder/und vom Träger getrennt in einem geeigneten Reaktionsgefäß erfolgen. Auf diese Weise kann die Menge der für die Gensynthese verfügbaren Nukleinsäurefragmente signifikant erhöht werden. Weiterhin werden die während der Synthese entstandenen verkürzten Fragmente, welche die Assemblierungsreaktion hemmen können, verdünnt und liegen im Vergleich zu den amplifizierten Volllängenfragmenten in vernachlässigbaren Konzentrationen vor. Damit kann auch die Erhöhung des Anteils an Volllängenfragmenten in einem für die Gensynthese vorgesehenen Gemisch durch die vorgeschaltete Amplifikation und damit Verdünnung von verkürzten Syntheseprodukten Bestandteil dieser Ausführungsform sein.

In noch einer weiteren Ausführungsform kann eine Isolierung von Nukleinsäurefragmenten mit der gewünschten korrekten Sequenz aus einem Gemisch von Nukleinsäuren erfolgen. Hierzu können beispielsweise bekannte Techniken, wie emulsionsbasierte PCR oder Einzelmolekülarrays, verwendet werden, bei denen klonale Molekülpopulationen oder Einzelmoleküle aus einem Gemisch von DNA-Fragmenten isoliert und sequenziert werden können. Durch Anwendung derartiger Verfahren während des Gensyntheseverfahrens kann das assemblierte Gen "monoklonalisiert" und jedes der einzelnen Fragmente separat sequenziert werden. Nach Sequenzverifikation kann das Fragment mit der gewünschten Sequenz identifiziert, isoliert und weiter prozessiert werden, z.B. durch Klonierung, DNA-basierende Assays, *in vitro* Proteinexpression etc. Das Gemisch von DNA-Fragmenten kann ein PCR-Produkt, ein Ligationsprodukt oder eine Oligonukleotidbibliothek sein.

Bekannt sind dem Fachmann Verfahren, bei denen eine emulsionsbasierte PCR gleichzeitig noch in den Micellen bzw. wässrigen Kompartimenten vorzugsweise jeweils ein Bead oder Partikel enthalten. Diese sind in besonders optimierten und für die Erfindung bevorzugten Varianten kleiner als 1 mm im Durchmesser. Bekannt ist z.B. das Verfahren der Firma 454, das die Sequenzierung von Abschnitten im Bereich 200 bis 300 Basen (Stand 2006) ermöglicht. Dabei wird DNA in kleinere Abschnitte fragmentiert und diese dann mit einheitlichen Linker- oder Adapter-Sequenzen durch Ligation ergänzt. Dieses Gemisch wird mit den beschriebenen Beads in einer Emulsions-PCR inkubiert. Die Primer für die PCR-Reaktion befinden sich auf den Beads als feste Phase. Als Reaktionsergebnis liegt eine Vielzahl von Beads vor, die jeweils klonal nur ein Fragment aus dem zuvor fragmentierten DNA-Material kovalent an der Oberfläche tragen. Im nächsten Schritt werden die Beads in einem Reaktionsträger immobilisiert, der zu den Beads und ihrer Größe passende Kavitäten enthält, und dann durch eine dem Fachmann bekannte so genannte Sequencing by synthesis Reaktion parallel die Sequenzen auf jedem der Beads erfasst.

In dem erfindungsgemäßen Verfahren können aus den Oligos aus der parallelen Synthese zunächst ein oder mehrere Assemblierungsreaktionen zusammengestellt werden. Die Zielsequenzen sind in einer Ausführungsform bei Nutzung der Emulsion-Bead-PCR entsprechend den Leseweiten der Sequenzierreaktion zu wählen und damit bevorzugt 10 bis 1.000 Nukleotide lang, besonders bevorzugt 40 bis 500. Der Vorteil dieser Ausführungsform liegt nun darin, ein Gemisch von assemblierten Sequenzen, die jeweils aus 2 oder mehr Oligos aufgebaut wurden und die möglicherweise Fehlstellen enthalten, in dem oben ausgeführten Verfahren auf den Beads zu klonalen Populationen zu amplifizieren und diese dann zu sequenzieren. Damit können in einer bevorzugten Ausführungsform Klonierung und Qualitätskontrolle der Zielsequenzen in einem Schritt zusammengefasst werden. Durch die Immobilisierung im Träger bei der Sequenzierung wird eine Lokalisation bewirkt. Es können diejenigen DNA- Zielsequenzen mit Sequenzen, die einem vorgegebenen Kriterium entsprechen, in einem nächsten Schritt entnommen werden. In einer weiteren bevorzugten Ausführungsform erfolgt eine Markierung durch ortsaufgelöste Zugabe eines Markers, z.B. eines spezifisch oder unspezifisch bindenden optischen Markers, wie z.B. einem Interkalator (Sybergreen).

Eine Ausführungsform umfasst ein Verfahren zur insbesondere parallelen Sequenzierung von mindestens einer Nukleinsäure in einem Gemisch umfassend assemblierte Nukleinsäuren, welche möglicherweise fehlerhafte Nukleotide enthalten, umfassend die Schritte:
(a) Amplifizieren eines Gemisches umfassend assemblierte Nukleinsäuren, die jeweils aus 2 oder mehr Nukleinsäurefragmenten aufgebaut sind, zu klonalen Populationen,
(b) Sequenzieren mindestens einer klonalen Populationen aus Schritt (a) und
(c) gegebenenfalls Isolieren mindestens einer Nukleinsäure, welche Fehler enthält, oder/und mindestens einer Nukleinsäure, welche korrekt ist.

Die Isolation erfolgt in einer bevorzugten Ausführungsform durch Isolation von einem oder mehreren der Beads. In einer alternativen Ausführungsform erfolgt die Isolation durch selektive Amplifikation mittels ortsaufgelöster Zugabe von PCR-Reagenzien. In einer alternativen Ausführungsform erfolgt eine Markierung durch ortsaufgelöste Zugabe eines Markers, z.B. eines spezifisch oder unspezifisch bindenden optischen Markers, wie z.B. eines Interkalators (Sybergreen), und anschließende Eluation mittels Laser Capture Methode, die dem Fachmann aus der Isolation einzelner Zellen bekannt ist.

Unerwünschte oder als fehlerhaft erkannte Klone (Beads) können physisch eliminiert werden. Dies kann in einer Ausführungsform durch selektive Behandlung mit einer starken Lichtquelle wie einem Laser geschehen. Alternativ kann eine weitere Immobilisierung oder Derivatisierung erfolgen, z.B. in einer lichtabhängigen Reaktion, z.B. eine Vernetzung, kovalente Modifikation oder die Anbindung eines Moleküls, das eine Extraktion oder Eliminierung unterstützt. So können Beads mit unerwünschter Sequenz bei der weiteren Verwertung des sequenzierten Produktes bzw. der Vielzahl an Produkten selektiv ausgeschlossen werden.

Die eluierten, isolierten oder anderweitig für weitere Schritte verfügbar gemachten erwünschten Zielsequenzen können ihrerseits zum Aufbau noch längerer Zielsequenzen eingesetzt werden. Sie können auch als Gemisch für nachfolgende Verfahrensschritte eingesetzt werden.

In einer Ausführungsform werden alle als notwendig erachteten Bestandteile eines Genoms in diesem Verfahren hergestellt. In einer bevorzugten Ausführungsform werden diese DNA-Abschnitte in einem nachfolgenden Schritt in einen Zielorganismus eingebracht, der daraus *in vivo* ein assembliertes Genom aufbaut. Ein besonders bevorzugter Zielorganismus ist Deinococcus radiodurans (auch als Micrococcus radiodurans bekannt), der sein eigenes Genom nach Fragmentierung, z.B. ionisierender Strahlung, in Fragmente kleiner als 10.000 Basen *in vivo* assemblieren kann.

Die Beads können isoliert oder im Sequzenzier-Reaktionsträger gelagert und zu einem späteren Zeitpunkt erneut eingesetzt werden.

Die Gewinnung der klonalen Sequenzen kann eine Ablösung oder eine Kopie ohne Zerstörung der kovalenten Anknüpfung an den Bead erfolgen. Mit Kopie ohne Zerstörung der kovalenten Anknüpfung an den Bead steht ein Bead später für eine erneute Gewinnung der klonalen Sequenzen zur Verfügung.

Parallele Sequenziermethoden wie die oben beschriebene eignen sich für die Überprüfung von Gemischen an Oligonukleotiden, wie sie als Teil der Erfindung und als Ausgangsmaterial für die Gensynthese beschrieben sind. In einer weiteren - gegebenenfalls unabhängigen - Ausführungsform wird die Zusammensetzung einer Bibliothek von Oligos aus einem parallelen Syntheseverfahren wie dem erfindungsgemäßen Verfahren in einem parallelen Sequenzierverfahren mit mindestens 100, vorzugsweise mit 1.000 bis 10.000, besonders bevorzugt mit 10.000 bis 100.000 und insbesondere mit 100.000 bis 100 Millionen parallelen Sequenzierreaktionen überprüft.

Dem Fachmann sind weitere Sequenzierverfahren bekannt, welche in der vorliegenden Erfindung eingesetzt werden können, so z.B. die Herstellung von so genannten Polonies als klonale DNA auf einem Reaktionsträger und nachfolgende Sequenzierung mittels Sequencing by synthesis Reaktion oder mittels Sequencing by ligation. Produkte, die diese Verfahren nutzen, sind u.a. erhältlich von der Firma Applied Biosystems (ABI, USA) unter dem Namen Solid sowie von der Firma Solexa/Illumina (USA). Eine spezielle Ausführungsform mit besonders sensitivem Nachweis ist das "true single molecule sequencing" (tSMS) von der Firma Helicos (USA), das ebenfalls parallel abläuft und somit ebenfalls für die Erfindung eingesetzt werden kann.

In Kombination mit der weiter oben ausgeführten Auswahl der Anzahl an Synthesekapazität, z.B. Belichtungspixeln, kann eine Steuerungs- oder Regelungslogik als Teil der Erfindung zum Einsatz kommen. Die hohe Parallelität der hier umfassten Sequenziermethoden erlaubt die Erfassung großer Datenmengen und damit die rationelle Anpassung von Syntheseparametern, um so den Anteil an verwertbaren Zielsequenzen anhand definierter Kriterien, z.B. Anteil korrekter Zielsequenzen, vorzunehmen.

Eine weitere Ausführungsformen der Erfindung betrifft die Erzeugung von Bibliotheken, die eine Vielzahl von Varianten eines Gens enthalten, durch Synthese multipler Varianten von einem oder mehreren der Nukleinsäurefragmente auf dem Träger vor der Genassemblierung.

Noch eine weitere Ausführungsform der Erfindung betrifft die enzymatische Abspaltung der Nukleinsäurefragmente vom Träger.

Noch eine weitere Ausführungsform der Erfindung betrifft die Aufreinigung einer Bibliothek von Nukleinsäurefragmenten durch Rehybridisierung auf einem Träger, welcher die komplementären Sequenzen enthält.

Noch eine weitere Ausführungsform der Erfindung betrifft die Anfügung primerspezifischer DNA-Sequenzen an die Nukleinsäurefragmente, welche das 5'-Ende und das 3'-Ende des zu synthetisierenden Nukleinsäuredoppelstrangs bilden. Auf diese Weise können mehrere aufeinander folgende Reaktionen mit dem gleichen Primerpaar durchgeführt werden. Außerdem können unterschiedliche Gene aus unterschiedlichen Trägern gleichzeitig mit denselben Primern amplifiziert werden, wodurch das Verfahren automatisiert wird.

Noch eine weitere Ausführungsform der Erfindung betrifft die Erzeugung synthetischer Targetnukleinsäuren für Standard-Mikroarrayanalyseverfahren, z.B. von Sonden, die auf Standard-Mikroarrays immobilisiert werden.

Die zuvor genannten Ausführungsformen können selbstverständlich miteinander kombiniert werden.

Generell können alle Reaktionsträger und festen Phasen für das erfindungsgemäße Verfahren genutzt werden, für die eine Synthese einer Matrix aus Nukleinsäurepolymeren möglich ist.

Dazu gehören als beispielhafte Vertreter die folgenden, dem Fachmann bekannten Reaktionsträger-Formate und festen Phasen:
- Flacher Reaktionsträger, auch "Chip" genannt,
- Poröser Träger,
- Reaktionsträger mit Elektroden,
- Reaktionsträger mit temporär oder permanent immobilisierter fester Phase aus Partikeln oder Beads,
- Mikrofluidischer Reaktionsträger,
- Oberflächen-Modifikation: Gele, Linker, Spacer, Polymere, amorphe Schichten, 3D-Matrizes.

Einige dieser Reaktionsträger können in Kombination verwendet werden, z.B. ein mikrofluidischer Reaktionsträger mit porösen Oberflächen.

Der Aufbau der DNA-Sonden findet vorzugsweise durch lichtgesteuerte *in situ* Synthese auf einem mikrofluidischen Träger, z.B. in einem GENIOM^{®} one Instrument (febit biotech GmbH) unter Verwendung geeigneter Schutzgruppenchemie in einer dreidimensionalen Mikrostruktur statt. In einem zyklischen Syntheseprozess wechseln sich Belichtungen und Kondensationen der Nukleotide so lange ab, bis an jeder Position des Arrays in den Mikrokanälen die gewünschte DNA-Sequenz vollständig aufgebaut wurde. Auf diese Weise können z.B. bis zu 48.000 Oligonukleotide mit einer Länge von z.B. bis zu 60 Einzelbausteinen hergestellt werden. Die Oligonukleotide können dabei kovalent an ein Spacermolekül, einen chemischen Abstandhalter auf der Glasoberfläche des Reaktionsträgers binden. Die Synthese verläuft softwaregesteuert und ermöglicht eine hohe Flexibilität beim Aufbau des Arrays, den der Anwender damit seinen Bedürfnissen entsprechend individuell konfigurieren kann. So können z.B. die Länge der Oligonukleotide, die Zahl der erzeugten Nukleinsäure-Sonden oder interne Kontrollen optimal auf das jeweilige Experiment abgestimmt werden.

In einer Ausführungsform werden qualitativ hochwertige und in der Sequenz frei programmierbare Nukleinsäuren in Form von Oligonukleotiden mit 10-200 Basen Länge kostengünstig und effizient in einer Vielfalt von 10 oder mehr unterschiedlichen Sequenzen bereitgestellt, um synthetische kodierende doppelsträngige DNA (synthetische Gene) herzustellen.

Der Aufbau von doppelsträngiger DNA aus Oligonukleotiden ist seit den 60er Jahren des letzten Jahrhunderts bekannt (Arbeiten von Khorana und anderen; siehe "Shabarova: Advanced Organic Chemistry of Nucleic Acids", VCH Weinheim). Er geschieht in der Mehrzahl der Fälle mit einem von zwei Verfahren (siehe Holowachuk et. al., PCR Methods and Applications, Cold Spring Harbor Laboratory Press).

Einmal erfolgt die Synthese des gesamten Doppelstrangs durch Synthese von einzelsträngigen Nukleinsäuren (geeigneter Sequenz), Aneinanderlagerung durch Hybridisieren komplementärer Bereiche dieser Einzelstränge und Verbinden des molekularen Rückgrats durch Enzyme, meist Ligase.

Demgegenüber gibt es auch die Möglichkeit einer Synthese von an den Rändern überlappenden Bereichen als einzelsträngige Nukleinsäuren, Aneinanderlagerung durch Hybridisieren, Auffüllen der einzelsträngigen Bereiche durch Enzyme (Polymerasen) und dann Verbinden des Rückgrats durch Enzyme, meist Ligase.

Ein bevorzugter Ablauf einer Gensynthese gemäß der Erfindung ist wie folgt: Allgemein wird im Rahmen eines modularen Systems eine Synthese vieler einzelner Nukleinsäurestränge durch Anwendung des erfindungsgemäßen Verfahrens zur hochparallelen DNA-Synthese auf Matrizenbasis durchgeführt. Es entstehen als Reaktionsprodukte Sets von Nukleinsäuren, die als Bausteine in einem nachgeschalteten Prozess dienen. Damit wird eine Sequenz-Matrix erzeugt, die über 100.000 unterschiedliche Sequenzen enthalten kann. Die Nukleinsäuren liegen in einzelsträngiger Form vor und können aus dem Träger eluiert werden oder direkt im Reaktionsträger zur Reaktion gebracht werden. Durch wiederholtes Kopieren in einem oder mehreren Arbeitsgängen kann die Matrize mehrfach abgeschrieben werden, ohne diese zu zerstören, und gleichzeitig wird eine Vermehrung der jeweiligen in der Matrix kodierten Sequenzen erreicht. Wie an anderer Stelle ausführlicher beschrieben, kann durch Kopieren von distal nach proximal dabei auch der Anteil an verkürzten Nukleinsäurepolymeren an der festen Phase ausgeblendet werden, wenn distal die Kopier-Initiationsstelle liegt. Eine Beispiel hierfür ist eine distal angefügte Promoter-Sequenz.

Der Träger mit der Matrix an festphasengebundenen Molekülen kann für spätere erneute Verwendung aufbewahrt werden. Somit wird in einer effizienten Weise die Vielfalt an Sequenzen, die in einem Reaktionsträger durch eine in situ Synthese erzeugt wurde, für weitere Prozessschritte zur Verfügung gestellt. Gleichzeitig kann durch das Design der Kopierreaktion eine hohe Qualität der abgeschriebenen Sequenzen erzielt werden.

Danach werden geeignete Kombinationen der abgelösten DNA-Stränge gebildet. Die Zusammenlagerung der einzelsträngigen Bausteine zu doppelsträngigen Bausteinen erfolgt innerhalb eines Reaktionsraums, der in einem einfachen Ansatz ein übliches Reaktionsgefäß, z.B. ein Plastikröhrchen, sein kann. In einer anderen bevorzugten Ausführungsform ist der Reaktionsraum Teil des Reaktionsträgers, der in einer Variante ein mikrofluidischer Reaktionsträger sein kann, in dem die notwendigen Reaktionen ablaufen. Ein weiterer Vorteil eines integrierten mikrofluidischen Reaktionsträgers ist die Möglichkeit der Integration weiterer Prozessschritte, wie z.B. eine Qualitätskontrolle durch optische Analytik. In einer Ausführungsform hat bereits die Synthese der Matrix in einem mikrofluidischen Träger stattgefunden, der dann gleichzeitig als Reaktionsraum für die nachgeschaltete Zusammenlagerung nutzbar ist.

Die Sequenz der einzelnen Bausteine wird dabei so gewählt, dass beim Inkontaktbringen der einzelnen Bausteine zueinander komplementäre Bereiche an den beiden zusammengebrachten Enden zur Verfügung stehen, um durch Hybridisierung dieser Bereiche eine spezifische Aneinanderlagerung von DNA-Strängen zu ermöglichen. Damit entstehen längere DNA-Hybride. Das Phosphodiester-Rückgrat des DNA-Moleküls wird durch Ligasen geschlossen. Sollten die Sequenzen so gewählt werden, dass in diesen Hybriden einzelsträngige Lücken bestehen, so werden diese Lücken in bekannter Vorgehensweise enzymatisch mittels Polymerasen aufgefüllt. (z.B. Klenow-Fragment oder Sequenase). So entstehen längere doppelsträngige DNA-Moleküle. Sollte es für die weitere Verwendung notwendig sein, diese verlängerten DNA-Stränge als Einzelstränge vorzulegen, so kann dies mit den dem Fachmann bekannten Verfahren zum Aufschmelzen von DNA-Doppelsträngen geschehen, wie z.B. Temperatur oder Alkali.

Durch die Zusammenführung von Clustern an derart synthetisierten DNA-Strängen innerhalb von Reaktionsräumen können wiederum längere Teilsequenzen des finalen DNA-Moleküls erzeugt werden. Dies kann stufenweise geschehen, und die Teilsequenzen werden dabei zu immer längeren DNA-Molekülen zusammengesetzt. Auf diese Weise lassen sich sehr lange DNA-Sequenzen als vollsynthetisches Molekül mit einer Länge von über 100.000 Basenpaaren erzeugen. Dies entspricht bereits dem Größenbereich eines Bacterial Artificial Chromosome BAC. Für den Aufbau einer Sequenz von 100.000 Basenpaaren aus überlappenden Bausteinen von 20 Nukleotiden Länge werden 10.000 individuelle Bausteine benötigt.

Dies kann mit den meisten der eingangs beschriebenen hochparallelen Synthese-Verfahren geleistet werden. Besonders bevorzugt sind für das erfindungsgemäße Verfahren dabei diejenigen Technologien, die den Array aus Nukleinsäurepolymeren in einer weitgehend frei programmierbaren Weise erzeugen und nicht auf das Einrichten von technischen Komponenten, wie z.B. photolithographischer Masken, angewiesen sind. Demnach sind besonders bevorzugte Ausführungsformen auf die projektorbasierte lichtgestützte Synthese, die indirekte projektorbasierte lichtgesteuerte Synthese mittels Photosäuren und Reaktionskammern in einem mikrofluidischen Reaktionsträger, die elektronisch induzierte Synthese mittels ortsaufgelöster Deprotektion an einzelnen Elektroden auf dem Träger und die fluidische Synthese mittels ortsaufgelöster Deposition der aktivierten Synthese-Monomere aufgebaut.

Für die rationelle Bearbeitung genetischer Moleküle und die systematische Erfassung aller möglichen Varianten müssen die Bausteine in ihrer individuellen Sequenz flexibel und ökonomisch hergestellt werden. Dies leistet das Verfahren durch den Einsatz einer programmierbaren Lichtquellenmatrix für die lichtabhängige ortsaufgelöste *in situ* Synthese der DNA-Stränge, die als Bausteine Verwendung finden. Diese flexible Synthese erlaubt die freie Programmierung der individuellen Sequenzen der Bausteine und damit auch die Erzeugung von beliebigen Varianten der Teilsequenzen oder der finalen Sequenz, ohne dass damit wesentliche Veränderungen von Komponenten des Systems (Hardware) verbunden wären. Nur durch diese programmierte Synthese der Bausteine und damit der finalen Syntheseprodukte kann die Vielfalt genetischer Elemente systematisch bearbeitet werden. Gleichzeitig erlaubt die Verwendung der computergesteuert programmierbaren Synthese die Automatisierung des gesamten Prozesses inklusive der Kommunikation mit entsprechenden Datenbanken.

Die Auswahl der Sequenz der einzelnen Bausteine kann bei Vorgabe der Zielsequenz rationell unter Berücksichtigung von biochemischen und funktionellen Parametern erfolgen. Dabei sucht ein Algorithmus nach Eingabe der Zielsequenz (z.B. aus einer Datenbank) die geeigneten Überlappungsbereiche heraus. Je nach der Aufgabenstellung können unterschiedlich viele Teilsequenzen erstellt werden, und zwar innerhalb eines zu belichtenden Reaktionsträgers oder auf mehrere Reaktionsträger verteilt. Die Anlagerungsbedingungen für die Bildung der Hybride, wie z.B. Temperatur, Salzkonzentration etc., werden durch einen entsprechenden Algorithmus auf die zur Verfügung stehenden Überlappungsbereiche abgestimmt. So wird eine maximale Spezifität der Aneinanderlagerung gewährleistet. Die Daten für die Zielsequenz können in einer vollautomatischen Version auch direkt aus öffentlichen oder privaten Datenbanken entnommen und in entsprechende Zielsequenzen umgewandelt werden. Die entstehenden Produkte können wiederum optional in entsprechend automatisierte Abläufe eingespeist werden, z.B. in die Klonierung in geeigneten Zielzellen.

Der stufenweise Aufbau durch Synthese der einzelnen DNA-Stränge in Reaktionsbereichen innerhalb umgrenzter Reaktionsräume erlaubt auch den Aufbau schwieriger Sequenzen, z.B. solche mit internen Wiederholungen von Sequenzabschnitten, wie sie z.B. bei Retroviren und entsprechenden retroviralen Vektoren vorkommen. Durch die Ablösung der Bausteine innerhalb der fluidischen Reaktionsräume kann eine Synthese beliebiger Sequenz erfolgen, ohne dass Probleme durch die Zuordnung der überlappenden Bereiche auf den einzelnen Bausteinen entstehen.

Die hohen Qualitätsanforderungen, die beim Aufbau sehr langer DNA-Moleküle notwendig sind, werden u.a. durch die Verwendung einer Echtzeit-Qualitätskontrolle erfüllt. Dabei wird die ortsaufgelöste Synthese der Bausteine überwacht, ebenso die Ablösung und die Zusammenlagerung bis hin zur Erstellung der finalen Sequenz. Dann laufen alle Prozesse in einem lichtdurchlässigen Reaktionsträger ab. Des Weiteren wird die Möglichkeit geschaffen, im Durchlicht Reaktionen und fluidische Vorgänge durch z.B. eine CCD-Detektion zu verfolgen.

Der miniaturisierte Reaktionsträger wird so ausgeführt, dass ein Ablösevorgang in den einzelnen Reaktionsräumen möglich ist, und somit die auf den innerhalb dieser Reaktionsräume liegenden Reaktionsbereichen synthetisierten DNA-Stränge in Clustern abgelöst werden. Bei geeigneter Ausführung des Reaktionsträgers ist die Zusammenlagerung der Bausteine in einem stufenweisen Prozess in Reaktionsräumen möglich, außerdem die Entnahme von Bausteinen, Teilsequenzen oder des finalen Produktes, oder auch die Sortierung bzw. Auftrennung der Moleküle.

Die Zielsequenz kann nach ihrer Fertigstellung als integriertes genetisches Element durch Transfer in Zellen eingebracht und dadurch kloniert und im Zuge funktioneller Studien untersucht werden. Eine weitere Möglichkeit ist es, das Syntheseprodukt zuerst weiter aufzureinigen oder zu analysieren, wobei diese Analyse z.B. eine Sequenzierung sein kann. Der Prozess der Sequenzierung kann auch durch direkte Kopplung mit einem entsprechenden Gerät beginnen, z.B. mit einer nach dem in der Patentanmeldung DE 199 24 327 arbeitenden Vorrichtung zur integrierten Synthese und Analyse von Polymeren. Es ist ebenfalls denkbar, die erzeugten Zielsequenzen nach der Klonierung zu isolieren und zu analysieren.

Das erfindungsgemäße Verfahren stellt über die damit erzeugten integrierten genetischen Elemente ein Werkzeug zur Verfügung, das für die Weiterentwicklung der molekularen Biologie die biologische Vielfalt in einem systematischen Prozess erfasst. Die Erzeugung von DNA-Molekülen mit gewünschter genetischer Information ist damit nicht mehr der Engpass molekularbiologischer Arbeiten, da von kleinen Plasmiden über komplexe Vektoren bis zu Mini-Chromosomen alle Moleküle synthetisch erzeugt werden können und für weitere Arbeiten zur Verfügung stehen.

Das Herstellungsverfahren erlaubt die parallele Erzeugung von zahlreichen Nukleinsäuremolekülen und damit einen systematischen Ansatz für Fragestellungen betreffend Regulationselemente, DNA-Bindestellen für Regulatoren, Signalkaskaden, Rezeptoren, Wirkung und Interaktionen von Wachstumsfaktoren etc.

Durch die Integration von genetischen Elementen in eine vollsynthetische Gesamt-Nukleinsäure können die bekannten genetischen Werkzeuge, wie Plasmide und Vektoren, weiter genutzt und es kann so auf die entsprechenden Erfahrungen aufgebaut werden. Andererseits werden sich diese Erfahrungen durch die angestrebte Optimierung der vorhandenen Vektoren etc. rasch verändern. Die Mechanismen, die z.B. ein Plasmid für die Propagation in einem bestimmten Zelltyp geeignet machen, lassen sich auf der Grundlage des erfindungsgemäßen Verfahrens erstmals rationell untersuchen.

Durch diese rationelle Untersuchung großer Varianten-Zahlen lässt sich der gesamte Kombinationsraum genetischer Elemente erschließen. Damit wird neben die zur Zeit in rascher Entwicklung befindliche hochparallele Analytik (u.a. auf DNA-Arrays oder DNA-Chips) als zweites wichtiges Element die programmierte Synthese integrierter genetischer Elemente geschaffen. Nur beide Elemente zusammen können das Fundament einer rationellen molekularen Biologie bilden.

Bei der programmierten Synthese von entsprechenden DNA-Molekülen ist nicht nur die beliebige Zusammensetzung der kodierenden Sequenzen und funktionellen Elemente möglich, sondern auch die Anpassung der Zwischenbereiche. Dies sollte rasch zu Minimal-Vektoren und MinimalGenomen führen, womit wiederum Vorteile durch die geringere Größe entstehen. Übertragungsvehikel, wie z.B. virale Vektoren, können dadurch effizienter gemacht werden, z.B. bei Verwendung von retroviralen oder adenoviralen Vektoren.

Über die Kombination bekannter genetischer Sequenzen hinaus ist die Entwicklung neuer genetischer Elemente möglich, die auf der Funktion vorhandener aufbauen kann. Gerade für solche Entwicklungsarbeiten ist die Flexibilität des Systems von enormem Wert.

Die synthetischen DNA-Moleküle sind dabei auf jeder Stufe der Entwicklung des hier beschriebenen Verfahrens voll kompatibel mit der vorhandenen Rekombinationstechnologie. Auch für "traditionelle" molekularbiologische Anwendungen können integrierte genetische Elemente bereitgestellt werden, z.B. durch entsprechende Vektoren. Der Einbau entsprechender Schnittstellen auch für bisher wenig verwendete Enzyme ist bei integrierten genetischen Elementen kein limitierender Faktor.

Ermöglicht wird mit diesem Verfahren die Integration aller gewünschten funktionellen Elemente als "genetische Module", wie z.B. Gene, Teile von Genen, Regulationselemente, virale Verpackungssignale etc., in das synthetisierte Nukleinsäuremolekül als Träger genetischer Information. Durch diese Integration ergeben sich u.a. folgende Vorteile:
Es können damit hochgradig funktionsintegrierte DNA-Moleküle entwickelt werden, wobei unnötige DNA-Bereiche wegfallen (Minimal-Gene, Minimal-Genome).

Die freie Kombination der genetischen Elemente sowie die Veränderungen der Sequenz, wie z.B. zur Anpassung an den exprimierenden Organismus/Zelltyp (Codonnutzung), werden ebenso ermöglicht wie Veränderungen der Sequenz zur Optimierung funktioneller genetischer Parameter, wie beispielsweise Genregulation.

Ebenfalls ermöglicht werden Veränderungen der Sequenz zur Optimierung funktioneller Parameter des Transkripts, z.B. Spleißen, Regulation auf mRNA-Ebene, Regulation auf Translationsebene, und darüber hinaus die Optimierung funktioneller Parameter des Genprodukts, wie z.B. die Aminosäuresequenz (z.B. Antikörper, Wachstumsfaktoren, Rezeptoren, Kanäle, Poren, Transporter etc.).

Darüber hinaus ist es möglich, Konstrukte zu erstellen, die über den RNAi-Mechanismus in die Genexpression eingreifen. Wenn solche Konstrukte für mehr als eine RNAi-Spezies kodieren, können in einem Multiplex-Ansatz mehrere Gene gleichzeitig inhibiert werden.

Insgesamt ist das mit dem Verfahren realisierte System extrem flexibel und erlaubt in bisher nicht vorhandener Weise die programmierte Erstellung von genetischem Material mit stark verringertem Aufwand an Zeit, Materialien und Arbeit.

Größere DNA-Moleküle, wie z.B. Chromosomen von mehreren hundert kbp, waren mit den vorhandenen Methoden nahezu nicht gezielt manipulierbar. Bereits komplexere (d.h. größere) virale Genome von mehr als 30 kbp (z.B. Adenoviren) sind mit den klassischen Methoden der Gentechnik schwierig zu handhaben und zu manipulieren.

Es kommt zu einer erhebliche Verkürzung bis zur letzten Stufe der Klonierung eines Gens: Das Gen oder die Gene werden als DNA-Molekül synthetisiert und dann (nach geeigneter Vorbereitung, wie Reinigung etc.) direkt in Zielzellen eingebracht und das Ergebnis studiert. Der mehrstufige, meist über Mikroorganismen, wie E.coli, laufende Klonierungsprozess (z.B. DNA-Isolation, Reinigung, Analyse, Rekombination, Klonierung in Bakterien, Isolation, Analyse etc.) wird damit auf die letzte Übertragung des DNA-Moleküls in die finalen Effektorzellen reduziert. Bei synthetisch hergestellten Genen oder Genfragmenten ist eine klonale Vermehrung in einem Zwischenwirt (zumeist E.coli) nicht mehr notwendig. Damit umgeht man die Gefahr, dass das für die Zielzelle bestimmte Genprodukt eine toxische Wirkung auf den Zwischenwirt ausübt. Damit hebt man sich deutlich ab von der Toxizität mancher Genprodukte, die bei der Verwendung klassischer Plasmid-Vektoren häufig zu erheblichen Problemen bei der Klonierung der entsprechenden Nukleinsäurefragmente führt.

Eine weitere erhebliche Verbesserung ist die Zeitverkürzung und die Reduktion der Arbeitsschritte, bis nach dem Sequenzieren von genetischem Material die dabei vorgefundenen potentiellen Gene als solche verifiziert und kloniert werden. Normalerweise werden nach Auffinden interessierender Muster, die als ORF in Frage kommen, mit Sonden (z.B. mittels PCR) in cDNA-Bibliotheken entsprechende Klone gesucht, die allerdings nicht die ganze Sequenz der ursprünglich bei ihrer Herstellung verwendeten Boten-RNA (messenger RNA, mRNA) enthalten müssen (Problem der "full lenght clones"). Bei anderen Verfahren wird mittels eines Antikörpers in einer Expressions-Genbibliothek gesucht (Screening). Beide Verfahren lassen sich mit dem erfahrungsgemäßen Verfahren extrem abkürzen: bei Vorliegen einer "in silico" bestimmten Gen-Sequenz (d.h. nach der Erkennung eines entsprechenden Musters in einer DNA Sequenz durch den Computer), oder nach Entschlüsselung einer Proteinsequenz, kann ein entsprechender Vektor mit der Sequenz oder Varianten davon direkt über programmierte Synthese eines integrierten genetischen Elementes erzeugt und in geeignete Zielzellen eingebracht werden.

Die so erfolgende Synthese von DNA-Molekülen bis zu mehreren 100 kbp erlaubt die direkte Komplett-Synthese von viralen Genomen, z.B. Adenoviren. Diese sind ein wichtiges Werkzeug in der Grundlagenforschung (u.a. Gentherapie), aber wegen der Größe ihres Genoms (ca. 40 kbp) schwer mit klassischen Methoden der Gentechnik zu handhaben. Besonders die rasche und ökonomische Erzeugung von Varianten zur Optimierung ist dadurch stark limitiert. Diese Limitierung wird durch das erfindungsgemäße Verfahren aufgehoben.

Durch das Verfahren erfolgen die Integration der Synthese, die Ablösung der Syntheseprodukte und die Zusammenlagerung zu einem DNA-Molekül in einem System. Mit Herstellungsverfahren der Mikrosystemtechnik können alle notwendigen Funktionen und Verfahrensschritte bis zur Aufreinigung des finalen Produktes in einem miniaturisierten Reaktionsträger integriert werden. Dies können Synthesebereiche, Ablösungsbereiche (Cluster), Reaktionsräume, Zuführungskanäle, Ventile, Pumpen, Konzentratoren, Auftrennungsbereiche etc. sein.

Plasmide und Expressionsvektoren können für sequenzierte Proteine oder entsprechende Teilsequenzen direkt hergestellt und die Produkte biochemisch und funktionell analysiert werden, z.B. unter Verwendung geeigneter Regulationselemente. Damit fällt die Suche nach Klonen in einer Gen-Bibliothek weg. Entsprechend können offene Leseraster ("open reading frames" ORF) aus Sequenzierarbeiten (z.B. Humangenomprojekt) direkt in entsprechende Vektoren programmiert und mit gewünschten genetischen Elementen kombiniert werden. Eine Identifikation von Klonen, z.B. in durch aufwendiges Screening von cDNA Bibliotheken, entfällt. Damit ist der Informationsfluss von der Sequenz-Analyse zur Funktions-Analyse stark verkürzt worden, denn am selben Tag, an dem ein ORF durch Analyse von Primärdaten im Computer vorliegt, kann ein entsprechender Vektor inklusive des vermuteten Gens synthetisiert und zur Verfügung gestellt werden.

Gegenüber konventioneller Festphasen-Synthese zur Gewinnung synthetischer DNA zeichnet sich das Verfahren gemäß Erfindung durch geringeren Materialaufwand aus. Zur Herstellung tausender von unterschiedlichen Bausteinen für die Erzeugung von einem komplexen integrierten genetischen Element mit mehreren 100.000 kbp Länge, in entsprechend parallelisiertem Format und bei entsprechender Miniaturisierung (siehe Ausführungsbeispiele), braucht ein mikrofluidisches System deutlich weniger Einsatzstoffe als ein konventioneller Festphasensynthese-Automat für einen einzelnen DNA-Oligomer (bei Verwendung einer einzigen Säule). Hier stehen Mikroliter dem Verbrauch von Millilitern gegenüber, d.h. ein Faktor von 1000.

Unter Berücksichtigung neuester Erkenntnisse der Immunologie erlaubt das vorgestellte Verfahren ein extrem rationelles und schnelles Impfstoff-Design (DNA-Vakzine).

## Patentansprüche

1. Verfahren zur Herstellung von synthetischen Nukleinsäuredoppelsträngen, umfassend die Schritte:
(a) Herstellen einer Vielzahl von unterschiedlichen Nukleinsäurefragmenten durch Festphasensynthese, vorzugsweise an verschiedenen Positionen eines gemeinsamen festen Trägers, und
(b) Zusammenfügen von mindestens zwei Nukleinsäurefragmenten aus (a) durch eine spezifische Hybridisierungsreaktion zwischen Überlappungsbereichen zueinander komplementärer Abschnitte der Nukleinsäurefragmente,
wobei mindestens einige Nukleinsäurefragmente, die einen AT-Gehalt größer als 50 % aufweisen, in einer gegenüber anderen Fragmenten gesteigerten Menge für Schritt (b) eingesetzt werden.

2. Verfahren gemäß Anspruch 1, wobei die Festphasensynthese mittels Licht gesteuert wird.

3. Verfahren gemäß Anspruch 1, wobei die Steuerung mittels Licht und einer programmierbaren Lichtquellenmatrix erfolgt.

4. Verfahren nach Anspruch 1, wobei die Festphasensynthese mittels einer Spotting-Methode erfolgt.

5. Verfahren gemäß Anspruch 1, wobei der Träger eine Vielzahl von immobilisierten Partikeln enthält.

6. Verfahren gemäß Anspruch 1, wobei Nukleinsäurefragmente als Primer für Primer Extension Methoden, Strand Displacement Amplifikation, Polymerase Chain Reaction, Site Directed Mutagenesis oder/und Rolling Circle Amplifikation oder/und Nukleinsäurefragmente für die Herstellung von synthetischen Genen, von Genfragmenten, von Gen-Clustern, von Gen-Fähren, von Gen-Vektoren, von Chromosomen, von Genomen, von optimierten Genomen, von minimalen Gen-Clustern, von minimalen Genomen oder/und von vollsynthetischen Genomen hergestellt werden.

7. Verfahren gemäß Anspruch 1, wobei Nukleinsäurefragmente als Mischungen mit gezielten oder/und randomisierten Varianten von synthetischen Genen, von Genfragmenten, von Gen-Clustern, von Gen-Fähren, von Gen-Vektoren, von Chromosomen, von Genomen, von optimierten Genomen, von minimalen Gen-Clustern, von minimalen Genomen und/oder von vollsynthetischen Genomen hergestellt werden.

8. Verfahren gemäß Anspruch 1, wobei die erzeugten Nukleinsäurefragmente oder/und die hergestellten synthetischen Nukleinsäuren eine Markierung für eine direkte oder indirekte Erfassung mit einem Messgerät, z.B. einem optischen, magnetischen, elektrischen oder/und chemilumineszenten Messgerät, oder/und Messverfahren erhalten.

9. Verfahren gemäß Anspruch 1, wobei die erzeugten Nukleinsäurefragmente oder/und die hergestellten synthetischen Nukleinsäuren eine Markierung für eine direkte oder indirekte Erfassung durch andere molekulare Strukturen wie z.B. Enzyme, Rezeptoren, Proteine erhalten.

10. Verfahren gemäß Anspruch 1, weiterhin umfassend die Isolation oder/und Extraktion der erzeugten Nukleinsäurefragmente oder/und der hergestellten synthetischen Nukleinsäuren oder/und von daraus aufgebauten kovalent oder/und nicht-kovalent verbundenen Hybridsträngen.

11. Verfahren gemäß Anspruch 1, weiterhin umfassend die Bindung der Fragmente, von daraus aufgebauten kovalent oder/und nicht-kovalent verbundenen Hybridsträngen oder/und der hergestellten synthetischen Nukleinsäuren mittels Haptene oder anderer in einer molekularen Erkennungsreaktion einsetzbaren Bausteine, einer funktionellen Gruppe oder/und einer Modifikation, die während einem der Schritte in die Nukleinsäuren eingebracht wird.

12. Verfahren gemäß Anspruch 1, weiterhin umfassend die Detektion der Fragmente, von daraus aufgebauten kovalent oder/und nicht-kovalent verbundenen Hybridsträngen oder/und der hergestellten synthetischen Nukleinsäuren.

13. Verfahren gemäß Anspruch 1, weiterhin umfassend den Abbau, die selektive Trennung, die Öffnung, die Degradation oder/und den enzymatischen Verdau der Fragmente, von daraus aufgebauten kovalent oder nicht-kovalent verbundenen Hybridsträngen oder/und der hergestellten synthetischen Nukleinsäuren.

14. Verfahren gemäß Anspruch 1, weiterhin umfassend die Anlagerung der Fragmente, von daraus aufgebauten kovalent oder/und nicht-kovalent verbundenen Hybridsträngen oder/und der hergestellten synthetischen Nukleinsäuren an eine Zielstruktur oder an Zielstrukturen in einem oder/und auf einem Reaktionsträger, auf einem Glas-Objektträger, in einem Reaktionsgefäß, an einer Zielorganelle, an einer Zielzelle, an einem Zielorgan, an einem Organismus, an einer Oberfläche, an einer biologischen Oberfläche, an einem molekularen Komplex, an einem Chromosom, an einem Viruspartikel oder/und an einem Proteinkomplex.

15. Verfahren gemäß Anspruch 1, weiterhin umfassend Amplifikationsschritte, bei denen die synthetisierten Nukleinsäurefragmente oder/und daraus gebildete doppelsträngige Zwischenprodukte einer Amplifikation, z.B. einer PCR, unterzogen werden.

16. Verfahren zur Herstellung eines Polypeptids, umfassend Synthese von synthetischen Nukleinsäuredoppelsträngen mit der das Polypeptid kodierenden Sequenz, umfassend die Schritte:
(a) Herstellen einer Vielzahl von unterschiedlichen Nukleinsäurefragmenten durch Festphasensynthese, vorzugsweise an verschiedenen Positionen eines gemeinsamen festen Trägers,
(b) Zusammenfügen von mindestens zwei Nukleinsäurefragmenten aus (a) durch eine spezifische Hybridisierungsreaktion zwischen Überlappungsbereichen zueinander komplementärer Abschnitte der Nukleinsäurefragmente,
wobei mindestens einige Nukleinsäurefragmente, die einen AT-Gehalt größer als 50 % aufweisen, in einer gegenüber anderen Fragmenten gesteigerten Menge für Schritt (b) eingesetzt werden, und wobei das Verfahren weiterhin umfasst: (c) Herstellung des Polypeptids unter Einsatz dieser Sequenz.

17. Verfahren nach Anspruch 1, wobei eine Abschrift der synthetischen Nukleinsäuren durch eine RNA-Polymerase erfolgt.

18. Verfahren nach Anspruch 17, wobei die so erzeugten Nukleinsäurefragmente für Genexpressions-Modulation mittels RNAi oder/und Antisense-Methoden eingesetzt werden.

19. Verfahren nach Anspruch 17, wobei die erzeugten Nukleinsäurefragmente für die Herstellung, Extraktion, Aufreinigung, Isolation oder/und Vorbereitung von Analyten oder/und Proben (sample preparation) eingesetzt werden.

## Claims

1. A method of preparation of synthetic nucleic acid double strands, comprising the steps:
(a) preparation of a multiplicity of different nucleic acid fragments by solid-phase synthesis, preferably at various positions of a common solid support, and
(b) joining of at least two nucleic acid fragments from (a) by a specific hybridization reaction between overlapping regions of mutually complementary segments of the nucleic acid fragments,
wherein at least some nucleic acid fragments having an AT content of more than 50% are used in an increased amount relative to other fragments for step (b).

2. The method according to claim 1, wherein the solid-phase synthesis is controlled by light.

3. The method according to claim 1, wherein control is by means of light and a programmable light source matrix.

4. The method according to claim 1, wherein the solid-phase synthesis takes place by a spotting method.

5. The method according to claim 1, wherein the support contains a multiplicity of immobilized particles.

6. The method according to claim 1, wherein nucleic acid fragments are prepared as primers for primer extension methods, strand displacement amplification, polymerase chain reaction, site directed mutagenesis or/and rolling circle amplification or/and nucleic acid fragments for the production of synthetic genes, of gene fragments, of gene clusters, of gene transfers, of gene vectors, of chromosomes, of genomes, of optimized genomes, of minimal gene clusters, of minimal genomes or/and of completely synthetic genomes.

7. The method according to claim 1, wherein nucleic acid fragments are prepared as mixtures with directed or/and randomized variants of synthetic genes, of gene fragments, of gene clusters, of gene transfers, of gene vectors, of chromosomes, of genomes, of optimized genomes, of minimal gene clusters, of minimal genomes and/or of completely synthetic genomes.

8. The method according to claim 1, wherein the nucleic acid fragments produced or/and the synthetic nucleic acids produced are labeled for direct or indirect detection with a measuring instrument, e.g. an optical, magnetic, electrical or/and chemiluminescent measuring instrument or/and method of measurement.

9. The method according to claim 1, wherein the nucleic acid fragments produced or/and the synthetic nucleic acids produced are labeled for direct or indirect detection through other molecular structures such as enzymes, receptors, proteins.

10. The method according to claim 1, further comprising the isolation or/and extraction of the nucleic acid fragments produced or/and the synthetic nucleic acids produced or/and of covalently or/and noncovalently bound hybrid strands constructed therefrom.

11. The method according to claim 1, further comprising the binding of the fragments, covalently or/and noncovalently bound hybrid strands constructed therefrom or/and the synthetic nucleic acids produced, by means of haptens or other building blocks that can be used in a molecular recognition reaction, of a functional group or/and of a modification, which is inserted in the nucleic acids during one of the steps.

12. The method according to claim 1, further comprising the detection of the fragments, covalently or/and noncovalently bound hybrid strands constructed therefrom or/and the synthetic nucleic acids produced.

13. The method according to claim 1, further comprising the decomposition, selective separation, opening, degradation or/and enzymatic digestion of the fragments, covalently or noncovalently bound hybrid strands constructed therefrom or/and the synthetic nucleic acids produced.

14. The method according to claim 1, further comprising the attachment of the fragments, covalently or/and noncovalently bound hybrid strands constructed therefrom or/and the synthetic nucleic acids produced, to a target structure or to target structures in or/and on a reaction support, on a glass microscope slide, in a reaction vessel, on a target organelle, on a target cell, on a target organ, on an organism, on a surface, on a biological surface, on a molecular complex, on a chromosome, on a virus particle or/and on a protein complex.

15. The method according to claim 1, further comprising amplification steps in which the synthesized nucleic acid fragments or/and double-stranded intermediates formed therefrom are submitted to amplification, e.g. PCR.

16. A method of preparation of a polypeptide, comprising the synthesis of synthetic nucleic acid double strands with the sequence encoding the polypeptide, comprising the steps:
(a) preparation of a multiplicity of different nucleic acid fragments by solid-phase synthesis, preferably at various positions of a common solid support, and
(b) joining of at least two nucleic acid fragments from (a) by a specific hybridization reaction between overlapping regions of mutually complementary segments of the nucleic acid fragments,
wherein at least some nucleic acid fragments having an AT content of more than 50% are used in an increased amount relative to other fragments for step (b), and wherein the method further comprises:
(c) production of the polypeptide using this sequence.

17. The method according to claim 1, wherein a copy of the synthetic nucleic acids is provided by an RNA polymerase.

18. The method according to claim 17, wherein the nucleic acid fragments produced in such a way are used for modulation of gene expression by means of RNAi or/and antisense methods.

19. The method according to claim 17, wherein the nucleic acid fragments produced are used for production, extraction, purification, isolation or/and preparation of analytes or/and samples (sample preparation).

## Revendications

1. Procédé de préparation d'acides nucléiques synthétiques double brin, comprenant les étapes de :
(a) préparation d'un certain nombre de différents fragments d'acide nucléique par synthèse en phase solide, de préférence en différentes positions d'un support solide commun, et
(b) assemblage d'au moins deux fragments d'acide nucléique de (a) par une réaction spécifique d'hybridation entre des domaines chevauchants de segments complémentaires l'un à l'autre des fragments d'acide nucléique,
où au moins quelques fragments d'acide nucléique, qui présentent une teneur en AT supérieure à 50%, sont mis en oeuvre pour l'étape (b), en une quantité augmentée par rapport aux autres fragments.

2. Procédé selon la revendication 1, où la synthèse en phase solide est contrôlée par la lumière.

3. Procédé selon la revendication 1, où le contrôle est réalisé par la lumière et une matrice programmable de source lumineuse.

4. Procédé selon la revendication 1, où la synthèse en phase solide est réalisée par un procédé de spotting.

5. Procédé selon la revendication 1, où le support contient une multitude de particules immobilisées.

6. Procédé selon la revendication 1, où les fragments d'acide nucléique sont préparés en tant qu'amorces pour les procédés d'extension par amorce, l'amplification par déplacement de brin, la réaction en chaine par polymérase, la mutagenèse dirigée et/ou l'amplification en cercle roulant, et/ou en tant que fragments d'acide nucléique pour la préparation de gènes synthétiques, de fragments de gène, de clusters de gènes, de vecteurs de gène, de chromosomes, de génomes, de génomes optimalisés, de clusters minimaux de gènes, de génomes minimaux et/ou de génomes complètement synthétiques.

7. Procédé selon la revendication 1, où les fragments d'acide nucléique sont préparés en tant que mélanges avec variantes ciblées et/ou statistiques de gènes synthétiques, de fragments de gène, de clusters de gènes, de vecteurs géniques, de vecteurs de gène, de chromosomes, de génomes, de génomes optimalisés, de clusters minimaux de gènes, de génomes minimaux et/ou de génomes complètement synthétiques.

8. Procédé selon la revendication 1, où les fragments d'acide nucléique produits et/ou les acides nucléiques synthétiques préparés reçoivent un marquage pour la détection directe ou indirecte avec un appareil de mesure, par exemple un appareil de mesure optique, magnétique, électrique et/ou chimio-luminescent, et/ou par un procédé de mesure.

9. Procédé selon la revendication 1, où les fragments d'acide nucléique produits et/ou les acides nucléiques synthétiques préparés reçoivent un marquage pour la détection directe ou indirecte par d'autres structures moléculaires comme par exemple, des enzymes, des récepteurs, des protéines.

10. Procédé selon la revendication 1, comprenant en outre, l'isolement et/ou l'extraction des fragments d'acide nucléique produits et/ou des acides nucléiques synthétiques préparés et/ou des brins hybrides liés de manière covalente et/ou non covalente, élaborés à partir de ceux-ci.

11. Procédé selon la revendication 1, comprenant en outre, la liaison des fragments, des brins hybrides liés de manière covalente et/ou non covalente, élaborés à partir de ceux-ci et/ou des acides nucléiques synthétiques préparés à l'aide d'haptène ou d'autres éléments pouvant être mis en oeuvre dans une réaction de reconnaissance moléculaire, un groupe fonctionnel et/ou une modification, qui est introduit dans les acides nucléiques au cours d'une étape.

12. Procédé selon la revendication 1, comprenant en outre, la détection des fragments, des brins hybrides liés de manière covalente et/ou non covalente, élaborés à partir de ceux-ci et/ou des acides nucléiques synthétiques préparés.

13. Procédé selon la revendication 1, comprenant en outre, la décomposition, la séparation sélective, l'ouverture, la dégradation et/ou la digestion enzymatique des fragments, des brins hybrides liés de manière covalente et/ou non covalente, élaborés à partir de ceux-ci et/ou des acides nucléiques synthétiques préparés.

14. Procédé selon la revendication 1, comprenant en outre, la fixation des fragments, des brins hybrides liés de manière covalente et/ou non covalente, élaborés à partir de ceux-ci et/ou des acides nucléiques synthétiques préparés, sur une structure cible ou sur des structures cibles dans un et/ou sur un support réactionnel, sur un porte-objet en verre, dans un récipient de réaction, sur un organite cible, sur une cellule cible, sur un organe cible, sur un organisme, sur une surface, sur une surface biologique, sur un complexe moléculaire, sur un chromosome, sur une particule virale et/ou sur un complexe protéique.

15. Procédé selon la revendication 1, comprenant en outre, des étapes d'amplification, dans lesquelles les fragments d'acide nucléique synthétisés et/ou les produits intermédiaires double brin formés à partir de ceux-ci sont soumis à une amplification, par exemple une PCR.

16. Procédé de préparation d'un polypeptide, comprenant la synthèse d'acides nucléiques double brin synthétiques avec une séquence codant le polypeptide, comprenant les étapes de :
(a) préparation d'une multitude de fragments d'acide nucléique différents par synthèse en phase solide, de préférence en différentes positions d'un support solide commun,
(b) assemblage d'au moins deux fragments d'acide nucléique de (a) par une réaction spécifique d'hybridation entre des domaines chevauchants de segments complémentaires l'un à l'autre des fragments d'acide nucléique,
où au moins quelques fragments d'acide nucléique, qui présentent une teneur en AT supérieure à 50%, sont mis en oeuvre pour l'étape (b), en une quantité augmentée par rapport aux autres fragments, et où le procédé comprend en outre :
(c) la préparation du polypeptide par utilisation de cette séquence.

17. Procédé selon la revendication 1, où une copie des acides nucléiques synthétiques est réalisée par une ARN polymérase.

18. Procédé selon la revendication 17, où les fragments d'acide nucléique ainsi produits sont mis en oeuvre pour la modulation de l'expression génique à l'aide d'un ARNi et/ou des procédés antisens.

19. Procédé selon la revendication 17, où les fragments d'acide nucléique produits sont mis en oeuvre pour la préparation, l'extraction, la purification, l'isolement et/ou le traitement préalable d'analytes et/ou de sondes (préparation d'échantillon).
